# EUROPÄISCHE PATENTSCHRIFT

(11) **EP 1 126 012 B1**
(45) Veröffentlichungstag und Bekanntmachung des Hinweises auf die Patenterteilung: **15.12.2004**
(21) Anmeldenummer: 01100239.1
(22) Anmeldetag: 03.01.2001
(51) Int. Cl.: C11D 3/48, C11D 3/33, C11D 1/72, C11D 1/62

(54) **Reinigungs- und Desinfektionssysteme für medizinische Instrumente**
Cleaning and sanitizing systems for medical devices
Systèmes détergents et désinfectants pour instruments médicaux

(30) Priorität: 17.02.2000 DE 10007324; 24.06.2000 DE 10030946
(43) Veröffentlichungstag der Anmeldung: 22.08.2001
(73) Patentinhaber: Bode Chemie GmbH & Co., 22525 Hamburg (DE)
(72) Erfinder: Albrecht, Harald, 95188 Issiggau (DE); Bloss, Richard, Dr., 25462 Rellingen (DE); Fehling, Thomas, 22305 Hamburg (DE); Knieler, Roland, Dr., 21228 Harmstorf (DE); Link, Monika, 22523 Hamburg (DE); Pietsch, Hanns, Dr., 20148 Hamburg (DE); Schulte-Schrepping, Dagmar, 25474 Hasloh (DE)

(56) Entgegenhaltungen:
- EP-A- 0 923 950
- EP-A- 0 969 080
- WO-A-96/23050
- WO-A-97/01624
- WO-A-98/22564
- DE-A- 19 603 977
- US-A- 3 812 046
- US-A- 5 403 505
- DATABASE WPI Section Ch, Week 199612 Derwent Publications Ltd., London, GB; Class A96, AN 1996-112632 XP002136348 & JP 08 012572 A (KOZAKAI YG), 16. Januar 1996 (1996-01-16)

## Beschreibung

Die vorliegende Erfindung betrifft flüssige Reinigungs- und Desinfektionssysteme mit einem Gehalt an Tensiden, Korrosionsschutzmitteln und weiteren Zusätzen, die die Reinigung verstärken sowie deren Verwendung zur Reinigung und gleichzeitigen oder nachfolgenden Desinfektion von medizinischen Instrumenten, insbesondere zur Reinigung und gleichzeitigen oder nachfolgenden Desinfektion von flexiblen Endoskopen.

Als Desinfektionsmittel werden Stoffe bezeichnet, die zur Desinfektion, d. h. zur Bekämpfung pathogener Mikroorganismen (z. B. Bakterien, Viren, Sporen, Klein- und Schimmelpilze) geeignet sind und zwar im allgemeinen durch Anwendung an der Oberfläche von Haut, Kleidung, Geräten, Räumen, aber auch von Trinkwasser, Nahrungsmitteln, Saatgut (Beizen) und als Bodendesinfektionsmittel.

Besonders lokal anzuwendende Desinfektionsmittel, z. B. zur Wunddesinfektion, werden auch als Antiseptika bezeichnet.

Desinfektionsmittel werden definiert als Stoffe oder Stoffgemische, die bei der Anwendung auf Gegenständen od. Oberflächen diese in einen Zustand versetzen, daß sie keine Infektion mehr verursachen. Ihre Wirkung muß bakterizid, fungizid, viruzid und sporizid (Sammelbegriff: mikrobizid) sein. Ein Effekt im Sinne der Bakteriostase ist für Desinfektionsmittel unzureichend. Sie sind daher im allgemeinen pantoxisch, d. h. sie entfalten ihre Wirkung gegen alle lebenden Zellen.

Je nach Verwendungszweck teilt man die Desinfektionsmittel ein in solche zur Wäsche-, Flächen-, Instrumenten-, Haut- und Hände- sowie zur Stuhl- und Sputumdesinfektion.

Unter Desinfektionsreiniger versteht man solche Desinfektionsmittel, die auch als Reinigungs- und gegebenenfalls Pflegemittel fungieren.

Unter Berücksichtigung der vielfältigen Forderungen, die an Desinfektionsmittel gestellt werden, wie z. B. breites Wirkungsspektrum, kurze Einwirkungszeiten, Hautverträglichkeit, geringe Toxizität, Materialverträglichkeit kommen nur einige Wirkstoff-Typen für den Einsatz in Betracht:
1. Die wichtigste Wirkstoff-Gruppe sind die Aldehyde (Formaldehyd, Glyoxal, Glutaraldehyd). Sie besitzen ein breites Wirkungsspektrum einschließlich Virus-Wirksamkeit und sporizider Wirkung bei Formaldehyd und Glutaraldehyd.
2. Phenol-Derivate besitzen eine gute bakterizide Wirkung, sind aber nicht sporizid. Gegenüber fast allen anderen Desinfektionsmittelwirkstoffen haben sie den Vorzug, durch Schmutz verhältnismäßig wenig beeinflußt zu werden. Sie eignen sich daher bes. zur Stuhldesinfektion. Typische Vertreter sind 2-Biphenylol und p-Chlor-m-kresol (4-Chlor-3-methylphenol).
3. Alkohole zeichnen sich durch schnelle Wirksamkeit aus, allerdings erst bei relativ hohen Konzentrationen von ca. 40-80%.
4. Die quaternären Ammonium-Verbindungen, Kationentenside (Invertseifen) und Ämphotenside gehören zur Klasse der Tenside. Sie zeichnen sich durch recht gute Haut- und Materialverträglichkeit sowie Geruchsneutralität aus. Ihr Wirkungsspektrum ist dagegen nur begrenzt. Hierher gehören z. B. Benzalkoniumchlorid, Cetrimoniumbromid, Cetylpyridiniumchlorid (Hexadecylpyridiniumchlorid) und andere.
   Quatemäre Ammoniumverbindungen sind organisch Ammoniumverbindungen mit quaternären Stickstoffatomen. Quaternäre Ammoniumverbindungen mit einem hydrophoben Alkyl-Rest sind biozid; ihr Einsatz ist freilich aus toxikologischen Gründen rückläufig.
   Quaternäre Ammoniumverbindungen werden durch Umsetzung tertiärer Amine mit Alkylierungsmitteln, wie z. B. Methylchlorid, Benzylchlorid, Dimethylsulfat, Dodecylbromid, aber auch Ethylenoxid hergestellt. In Abhängigkeit von dem eingesetzten tert. Amin unterscheidet man drei Gruppen: 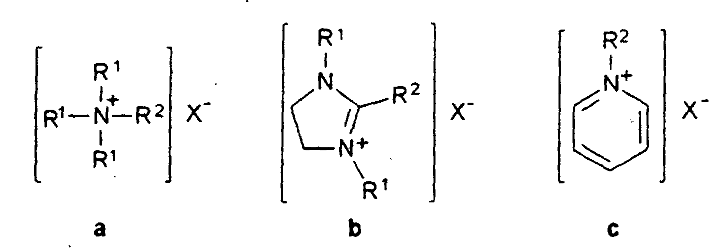
   a) Lineare Alkylammoniumverbindungen
   b) Imidazoliniumverbindungen
   c) Pyridinium-Verb. R¹ = CH₃, R² = C₈₋₁₈, X = Halogen.

   Die Alkylierung tertiärer Amine mit einem langen Alkylrest und zwei Methylgruppen gelingt besonders leicht, auch die Quaternierung tertiärer Amine mit zwei langen Resten und einer Methylgruppe kann mit Hilfe von Methylchlorid unter milden Bedingungen durchgeführt werden. Amine, die über drei lange Alkylreste oder hydroxysubstituierte Alkylreste verfügen, sind wenig reaktiv und werden bevorzugt mit Dimethylsulfat quaterniert.
5. Von den Halogenen besitzen Chlor und lod eine gewisse Bedeutung als Desinfektionsmittel. Chlor ist von der Wasseraufbereitung und Schwimmbaddesinfizierung her bekannt und damit seine unangenehmen Eigenschaften wie Geruch und Korrosivität. Trotz der ausgezeichneten Wirkung gegen Bakterien, Pilze, Sporen und Viren haben chlorhaltige Desinfektionsmittel im Humanbereich aus den obengenannten Gründen und wegen der starken Chlor-Zehrung durch organ. Substanzen keine starke Verbreitung gefunden. Dagegen werden Hypochlorite, Chlorkalk- und Chlorisocyanursäuren als technische Desinfektionsmittel noch umfänglich benutzt. lodtinktur wird im medizinischen Bereich als Antiseptikum verwendet.
6. Desinfektionsmittel auf Basis von aktivem Sauerstoff (z. B. Wasserstoffperoxid, Peroxyessigsäure) haben in letzter Zeit wieder etwas an Bedeutung gewonnen.

Außer den genannten Mikrobizid-Wirkstoffen sind noch eine Anzahl von mikrobistat. Substanzen und Konservierungsmitteln (Diphenylether, Carbanilide, Acetanilide aromatischen Säuren und deren Salze) für spezifische Verwendung auf dem Markt, die im erweiterten Sinne den Desinfektionsmitteln zugerechnet werden.

Eine einheitliche Wirkungsweise der Desinfektionsmittel ist nicht zu erkennen. Während manche Präpärate auf die Cytoplasmamembran der Bakterien zerstörend wirken sollen, wird von anderen eine irreversible Blockierung wichtiger Sulfidbindungen bei Enzymen oder von Spurenelementen (durch Chelatisierung) angenommen.

Endoskope sind röhren- oder schlauchförmige Instrumente, welche zur Untersuchung von Körperhohlräumen und Hohlorganen verwendet werden. Sie sind mit einem optischen System, bestehend aus Objektiv und Okular, einer Beleuchtungseinrichtung und meist Spül- und Absaugvorrichtungen sowie Kanälen zum Einführen spezieller Instrumente ausgestattet. Flexible Endoskope (welche auch als Fiberendoskope bezeichnet werden) sind mit einem Glasfaserlichtleiter ausgerüstet, welcher aus einem dicht gepackten Bündel aus haarfeinen und deshalb hochflexiblen Einzelfasem besteht. Durch die Flexibilität der Glasfasern können Licht und Bild über praktisch jede beliebige Abwinklung übertragen werden. Zusätzlich zu den Faserbündeln und den Bowdenzügen für die Bewegung der Spitze sind Kanäle für Wasserspülung und Luft sowie ein in der Regel größerer Biopsie- und gleichzeitig Absaugkanal untergebracht.

Die Endoskopie kann allein, beispielsweise zur Entnahme von Gewebeproben, aber auch in Kombination mit weiteren Methoden, wie z. B. der Ultraschalldiagnostik (Endosonographie) oder der Röntgendiagnostik durchgeführt werden. Gebräuchlich sind beispielsweise Röntgenkontrastdarstellungen der Gallenblase und der Gallengänge sowie des Pankreasgangsystems. Hierbei wird - z. B. im Rahmen einer endoskopischen retrograden Cholangiographie (ERC) bzw. einer endoskopischen retrograden Cholangiopankreatikographie (ERCP) - ein Röntgenkontrastmittel unter Röntgenkontrolle in das zu untersuchende Gangsystem eingebracht.

Röntgenkontrastmittel sind Mittel zur Verbesserung der röntgenographischen Darstellung von Körperräumen, Hohlorganen und Gefäßen. Sie absorbieren Röntgenstrahlung entweder schwächer (negative Röntgenkontrastmittel) oder stärker (positive Röntgenkontrastmittel) als das umgebende Körpergewebe. Die radioopaken (d. h. strahlungsundurchlässigen) positiven Röntgenkontrastmittel enthalten vorwiegend Elemente hoher Ordnungszahlen (wie lod und Barium), da deren Absorptionsvermögen für Röntgenstrahlen größer ist. Für die Cholangiographie werden in der Regel organische Iod-Derivate, die sich von Pyridinen oder aromatischen Carbonsäuren ableiten, verwendet. Beispiele hierfür sind lodoxaminsäure [3,3'-(4,7,10,13-Tetraoxahexadecandioyldiamino)-bis-(24,6-triiodbenzoesäure)] und lotroxinsäure [33'-(3,6,9-Trioxaundecandioyldiamino)bis-(2,4,6-triiodbenzoesäure)].

Endoskopien finden zum Großteil in mikrobiell besiedelten Körperbereichen statt. Die Anzahl endoskopischer Untersuchungen des oberen und unteren Gastrointestinaltraktes und des Tracheobronchialsystems, häufig verbunden mit invasiven Eingriffen, ist sehr hoch. Eine Übertragung von Krankheitserregern durch kontaminierte Endoskopien selbst und durch endoskopisches Zubehör konnte vielfach belegt werden.

Insbesondere Infektionen mit langer Inkubationszeit sind nur sehr schwer und in der Praxis meist gar nicht zu erfassen. Ferner muß damit gerechnet werden, daß auch Patienten, deren Erkrankung stumm oder unerkannt ist, mit Endoskopen in Berührung kommen. Besonderes Gewicht haben in diesem Zusammenhang Infektionen mit Mikroorganismen, deren Übertragung durch das Blut und über die Schleimhäute erfolgt, wie z. B. Tuberkulose, Hepatitis, HIV-Infektionen, Salmonellose, Yersinien-Infektionen, Ruhr und dergleichen mehr.

Eine Anforderung an die Endoskopie sollte daher die uneingeschränkte Einhaltung aller erforderlichen Schutzmaßnahmen zur Infektionsverhütung sein. Allerdings war die Reinigung und Desinfektion der Fiberendoskope lange Zeit ein Stiefkind der technologischen Entwicklung. So gibt es beispielsweise Probleme mit Restwasser und entsprechender bakterieller Fehlbesiedlung, darüberhinaus lassen sich Endoskope nur schwer trocknen. Daher muß im Vorfeld einer endoskopischen Untersuchung nicht nur die Übertragung von Mikroorganismen, die von Patienten stammen, verhindert werden, sondern es sollte ferner ein besonderes Augenmerk auf Krankheitserreger aus Spülwasser oder stagnierendem Wasser gerichtet werden.

Der Stand der Technik kennt flüssige Desinfektionsreiniger zur Reinigung und Desinfektion von flexiblen Endoskopen, welche neben Tensiden und Desinfektionswirkstoffen im wesentlichen organische Lösemittel, Säuren oder Laugen sowie teilweise auch Enzyme enthalten. Allerdings weisen die genannten Verbindungen einige Nachteile auf.

Lösemittel, Säuren und Laugen sind zwar von ihrer Wirksamkeit her zur Reinigung prinzipiell geeignet, haben aber den Nachteil, daß sie für das empfindliche Material zu aggressiv sind und die Endoskope dementsprechend beschädigen können.

Die flüssigen Desinfektionsreiniger des Standes der Technik sind bei der Entfernung von Blut- und Sekretresten sowie bei der Ablösung von Fett und anderen kohlenwasserstoffhaltigen Verbindungen relativ gut wirksam. Allerdings eignen sie sich nicht, um Reste von Röntgenkontrastmitteln vollständig zu entfernen.

Bereits geringste Mengen zurückgebliebenes Röntgenkontrastmittel schließen Bakterien in den Endoskopkanälen ein, die mit den üblichen Desinfektionsmitteln dann nicht mehr vollständig beseitigt werden können. Bei regelmäßigem Einsatz flexibler Endoskope in der Praxis baut sich so im Laufe der Zeit auf der Innenseite der Kanäle ein zunehmender Film des Röntgenkontrastmittels und weiterer Rückstände auf, was zur Folge haben kann, daß das mikrobiologische Ergebnis selbst nach der an die Reinigung anschließenden Desinfektion nicht mehr befriedigend ist.

Für die Patienten bedeutet dies ein erhöhtes Infektionsrisiko und stellt dementsprechend eine erhebliche Gefährdung dar. Zum Schutz vor Infektionen bleibt dann nur noch die Möglichkeit einer Reparatur, was zum einen aufwendig und zum anderen teuer ist.

Aufgabe der Erfindung war es daher, ein flüssiges reinigendes und desinfizierendes System zu entwickeln, das die Nachteile des Standes der Technik nicht aufweist, welches also eine gute Reinigungsleistung und gleichzeitige oder nachfolgende Desinfektion bei zugleich hoher Materialverträglichkeit zeigt und sich auch zur Entfernung von Röntgenkontrastmitteln, wie z. B. organischen, aromatischen lod-Derivaten, insbesondere aus flexiblen Endoskopen eignet.

Es wurde nun überraschend gefunden, daß alle diese Aufgaben gelöst werden durch ein reinigendes und desinfizierendes System für medizinische Instrumente, dadurch gekennzeichnet, daß es
■ mindestens ein nichtionisches Tensid und
■ Pyrrolidoncarbonsäure
■ sowie mindestens ein desinfizierendes Agens
umfaßt.

Es war insbesondere überraschend, daß die erfindungsgemäßen reinigenden und desinfizierenden Systeme besser und schonender reinigen als die Zubereitungen des Standes der Technik und daß durch diese verbesserte Reinigung und gleichzeitige oder nachfolgenden Desinfektion bei den überprüften flexiblen Endoskopen das mikrobiologische Problem des Standes der Technik wesentlich verringert werden konnte.

Erstaunlicherweise lassen sich mit den erfindungsgemäßen reinigenden und desinfizierenden Systemen auch angetrocknete schwerlösliche aromatische Halogenverbindungen, wie z. B. Röntgenkontrastmittelrückstände, vollständig von den Oberflächen ablösen, bei gleichzeitiger oder auch nachfolgender Desinfektion.

Durch den Einsatz der erfindungsgemäßen reinigenden und desinfizierenden Systeme sind flexible Endoskope auch nach Monaten ständigen Einsatzes in der Praxis mikrobiologisch immer noch einwandfrei. Dadurch wird die Sicherheit der Patienten wesentlich erhöht und die Lebensdauer dieser hochwertigen medizinischen Geräte verlängert.

So konnte beispielsweise nachgewiesen werden, daß die erfindungsgemäßen reinigenden und desinfizierenden Systeme besonders bei Duoendoskopen, über die Röntgenkontrastmittel bevorzugt verabreicht werden, eine im Vergleich zu den Systemen des Standes der Technik wesentlich bessere Reinigungs- und Desinfektionsleistung haben. Hierdurch konnte der mikrobiologische Status der Duoendoskope (nach anschließender Desinfektion) stark verbessert werden.

Gegenstand der Erfindung ist dementsprechend auch die Verwendung von reinigenden und desinfizierenden Systemen, welche
■ mindestens ein nichtionisches Tensid und
■ Pyrrolidoncarbonsäure
■ sowie mindestens ein desinfizierendes Agens
enthalten, zur Reinigung und gleichzeitiger oder nachfolgender Desinfektion empfindlicher medizinischer Instrumente, flexibler Endoskope und empfindlicher medizinischer Oberflächen.

Erfindungsgemäß vorteilhaft werden das oder die nichtionische Tenside gewählt aus der Gruppe der Alkylethoxylate, deren Alkylgruppe eine gesättigte oder ungesättigte, geradoder verzweigtkettige Alkylgruppe mit 10 bis 18, vorzugsweise 12 bis 14 Kohlenstoffatomen ist, wobei sie vorzugsweise pro Molekül 2 bis 15, insbesondere 5 bis 9, speziell 7 Ethylenoxideinheiten enthalten. Ganz besonders bevorzugt sind Isotridecanolethoxylat und/oder Fettalkoholpolyglykolether.

Vorteilhaft wird die Gesamtmenge an nichtionischen Tensiden (eine oder mehrere Verbindungen) aus dem Bereich von 1,0 bis 20,0 Gew.-%, vorzugsweise von 5,0 bis 15,0 Gew.-% gewählt, jeweils bezogen auf das Gesamtgewicht der Zubereitungen.

Erfindungsgemäß wird die Pyrrolidoncarbonsäure (Pyroglutaminsäure), welche sich durch die folgende Strukturformel auszeichnet: 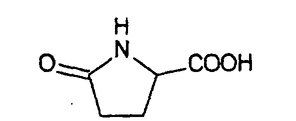 eingesetzt.

Vorteilhaft wird die Gesamtmenge der Aminosäure aus dem Bereich von 0,1 bis 10,0 Gew.-%, vorzugsweise von 0,5 bis 2,0 Gew.-% gewählt, jeweils bezogen auf das Gesamtgewicht der Zubereitungen.

Das oder die desinfizierenden Agentien werden bevorzugt gewählt aus der Gruppe der Aldehyde (z.B. Formaldehyd, Glyoxal, Glutaraldehyd), der Phenol-Derivate (z.B. 2-Biphenylol und p-Chlor-m-kresol (4-Chlor-3-methylphenol), der Alkohole, der quatemären Ammonium-Verbindungen (z. B. Benzalkoniumchlorid. Cetrimoniumbromid. Cetylpyridiniumchlorid (Hexadecylpyridiniumchlorid). Aldehyde und quaternäre Ammoniumverbindungen sind dabei ganz besonders bevorzugt.

In einer besonders vorteilhaften Ausführungsform können die erfindungsgemäßen reinigenden und desinfizierenden Systeme ferner Amphotenside enthalten. Amphotenside sind Tenside, die sowohl saure als auch basische hydrophile Gruppen besitzen und sich also je nach Bedingung sauer oder basisch verhalten. Vorteilhaft im Sinne der vorliegenden Erfindung sind beispielsweise Amphotenside auf der Basis von aliphatischen Polyaminen mit Carboxy-, Sulfo- oder Phosphono-Seitenketten, wie beispielsweise R-NH-(CH₂)ₙ-COOH.

Erfindungsgemäß bevorzugt sind z. B. Amphotenside, deren Alkylgruppe eine gesättigte oder ungesättigte, gerad- oder verzweigtkettige Alkylgruppe mit 10 bis 18, vorzugsweise 12 bis 14 Kohlenstoffatomen ist.

Insbesondere vorteilhaft sind ferner Amphotenside aus der Gruppe der Amphopropionate, wie z. B. das Cocobetainamido Amphopropionat, welches sich durch die folgende Struktur auszeichnet:

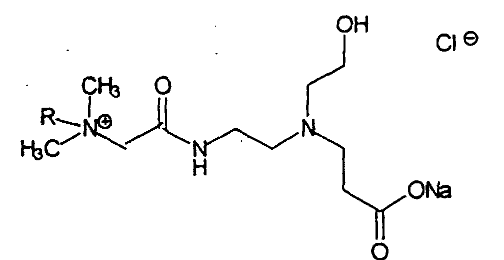

Vorteilhaft wird die Gesamtmenge an Amphotensiden (eine oder mehrere Verbindungen) aus dem Bereich von 1,0 bis 10,0 Gew.-%, vorzugsweise von 2,0 bis 5,0 Gew.-% gewählt, jeweils bezogen auf das Gesamtgewicht der Zubereitungen.

Die erfindungsgemäßen reinigenden und desinfizierenden Systeme können in Form von flüssigen Konzentraten vorliegen und beispielsweise in Dosierbeutel abgepackt werden, so daß eine Gebrauchslösung jeweils frisch angesetzt werden kann. Es ist insbesondere vorteilhaft, wenn der pH-Wert des Konzentrates zwischen 4 und 10 liegt. Die flüssigen Konzentrate werden vorzugsweise mit Wasser zu einer Gebrauchslösung verdünnt. Bevorzugt sind Gebrauchslösungen, die 1,0 bis 5 Gew.-% an erfindungsgemäßen reinigenden und desinfizierenden Systeme enthalten. Die Gebrauchslösung hat vorzugsweise einen pH-Wert zwischen 5 und 9, um eine optimale Schonung des empfindlichen Materials zu erreichen.

Vorteilhaft ist es, die Verdünnung so durchzuführen, daß der Gehalt der einzelnen Substanzen in der Gebrauchslösung wie folgt ist:
■ nichtionische Tenside: zwischen 0,005 und 1 Gew.-%
■ Aminosäure: zwischen 0,0005 und 0,5 Gew.-%
■ gegebenenfalls Amphotenside: zwischen 0,005 und 0,5 Gew.-%
■ desinfizierende Agentien: zwischen 0,1 und 2,0 Gew.-%

Die erfindungsgemäßen reinigenden und desinfizierenden Systeme können ferner vorteilhaft zusätzlich übliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können. Es wird bevorzugt, den Gehalt an einem oder mehreren Korrosionsinhibitoren zwischen 0,1 und 5 Gew.-%, bezogen auf das Gesamtgewicht der Formulierung, zu wählen.

Die erfindungsgemäßen reinigenden und desinfizierenden Systeme können darüberhinaus vorteilhaft zusätzlich Komplexbildner und/oder pH-Regulatoren enthalten, um den negativen Einfluß schwankender Wasserhärte zu reduzieren bzw. um zu gewährleisten, daß der pH-Wert des reinigenden und desinfizierenden Systems in einem Bereich liegt, der das empfindliche Material nicht schädigt.

Zusätzlich zu den vorstehend genannten Komponenten können die erfindungsgemäßen reinigenden und desinfizierenden Systeme für derartige Zubereitungen übliche Konservierungsstoffe, Farbstoffe, Duftstoffe und/oder andere übliche Hilfsstoffe enthalten. Diese tragen in der Mehrzahl der Fälle nicht zur reinigenden Wirkung bei, sondern dienen der Lagerbarkeit sowie ästhetischen Zwecken. Es ist jedoch auch möglich, solche Komponenten zu verwenden, die eine (konservierende, pflegende) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Es kann auch von Vorteil sein, die erfindungsgemäßen reinigenden und desinfizierenden Systeme so auszugestalten, daß sie
A) eine erste Zubereitung in Form eines flüssigen Reinigungsmittels für medizinische Instrumente, welches
   ■ mindestens ein nichtionisches Tensid und
   ■ Pyrrolidoncarbonsäure
   enthält,
   und
B) eine zweite Zubereitung, welche in einem geeigneten Träger
   ■ mindestens ein desinfizierendes Agens
enthält,
umfassen.

Ein derart ausgestaltetes erfindungsgemäßes reinigendes und desinfizierendes System wird beispielsweise vorteilhaft in Form eines "Sets" verwirklicht, bei welchem die Zubereitungen A) und B) getrennt voneinander, in verschiedenen Behältern aufbewahrt werden.

Die Zubereitungen A) können in einem solchen Falle vorteilhaft so ausgestaltet werden, daß das oder die nichtionische Tenside gewählt werden aus der Gruppe der Alkylethoxylate, deren Alkylgruppe eine gesättigte oder ungesättigte, gerad- oder verzweigtkettige Alkylgruppe mit 10 bis 18, vorzugsweise 12 bis 14 Kohlenstoffatomen ist, wobei sie vorzugsweise pro Molekül 2 bis 15, insbesondere 5 bis 9, speziell 7 Ethylenoxideinheiten enthalten. Ganz besonders bevorzugt sind Isotridecanolethoxylat und/oder Fettalkoholpolyglykolether.

Vorteilhaft wird die Gesamtmenge an nichtionischen Tensiden (eine oder mehrere Verbindungen) aus dem Bereich von 1,0 bis 20,0 Gew.-%, vorzugsweise von 10,0 bis 15,0 Gew.-% gewählt, jeweils bezogen auf das Gesamtgewicht der Zubereitungen.

Vorteilhaft wird die Gesamtmenge der Aminosäure aus dem Bereich von 0,1 bis 10,0 Gew.-%, vorzugsweise von 0,5 bis 2,0 Gew.-% gewählt, jeweils bezogen auf das Gesamtgewicht der Zubereitungen.

In einer besonders vorteilhaften Ausführungsform können die Zubereitungen A ferner Amphotenside enthalten, bevorzugt einer Gesamtmenge an Amphotensiden (eine oder mehrere Verbindungen) aus dem Bereich von 1,0 bis 10,0 Gew.-%, vorzugsweise von 2,0 bis 5,0 Gew.-% gewählt, jeweils bezogen auf das Gesamtgewicht der Zubereitungen.

Die Zubereitungen A) können in Form von flüssigen Konzentraten vorliegen und beispielsweise in Dosierbeutel abgepackt werden, so daß eine Gebrauchslösung jeweils frisch angesetzt werden kann. Es ist insbesondere vorteilhaft, wenn der pH-Wert des Konzentrates zwischen 5 und 9 liegt. Die flüssigen Konzentrate werden vorzugsweise mit Wasser zu einer Gebrauchslösung verdünnt. Bevorzugt sind Gebrauchslösungen, die 0,5 bis 5 Gew.-% an erfindungsgemäßen reinigenden und desinfizierenden Systeme enthalten. Die Gebrauchslösung hat vorzugsweise einen pH-Wert zwischen 6 und 8, um eine optimale Schonung des empfindlichen Materials zu erreichen.

Vorteilhaft ist es, die Verdünnung so durchzuführen, daß der Gehalt der einzelnen Substanzen in der Gebrauchslösung wie folgt ist:
■ nichtionische Tenside: zwischen 0,005 und 1 Gew.-%
■ Aminosäure: zwischen 0,0005 und 0,5 Gew.-%
■ gegebenenfalls Amphotenside: zwischen 0,005 und 0,5 Gew.-%

Die Zubereitungen A) können ferner vorteilhaft zusätzlich übliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können. Es wird bevorzugt, den Gehalt an einem oder mehreren Korrosionsinhibitoren zwischen 0,1 und 5 Gew.-%, bezogen auf das Gesamtgewicht der Formulierung, zu wählen.

Die Zubereitungen A) können darüberhinaus vorteilhaft zusätzlich Komplexbildner und/oder pH-Regulatoren enthalten, um den negativen Einfluß schwankender Wasserhärte zu reduzieren bzw. um zu gewährleisten, daß der pH-Wert des reinigenden und desinfizierenden Systems in einem Bereich liegt, der das empfindliche Material nicht schädigt.

Zusätzlich zu den vorstehend genannten Komponenten können die Zubereitungen A) für derartige Zubereitungen übliche Konservierungsstoffe, Farbstoffe, Duftstoffe und/oder andere übliche Hilfsstoffe enthalten.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die Zubereitungen B) enthalten ein oder mehrere desinfizierende Agentien in einem geeigneten Träger. Sie können in Form von festen Konzentraten, z. B. in (Brause-) Tabletten-, Pulver-, Pellet- oder Granulatform, vorliegen und beispielsweise in Dosierbeutel abgepackt werden, so daß eine Gebrauchslösung jeweils frisch angesetzt werden kann. Sie können aber auch in Form von Lösungen oder Suspensionen in nicht-wäßrigen Lösungsmitteln vorliegen.

Bevorzugt sind Zubereitungen B), welche auch Tenside enthalten, wie sie üblicherweise Verwendung finden, beispielsweise Sulfobetaine, Alkylsulfonate, Alkylbenzolsulfonate und/oder nichtionische Tenside auf Basis von Ethylenoxid und/oder Butylenoxid.

Ferner können die Zubereitungen B) vorteilhaft zusätzlich übliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können.

Zusätzlich zu den vorstehend genannten Komponenten können die Zubereitungen B) für derartige Zubereitungen übliche Farbstoffe, Duftstoffe und/oder andere übliche Hilfsstoffe, wie beispielsweise Dickungs-, Granulierungs-, Tablettierungs- oder Dispergierhilfsmittel, enthalten. Diese tragen in der Mehrzahl der Fälle nicht zur desinfizierenden. konservierenden oder antiseptischen Wirkung bei, sondern dienen der Handhabbar- bzw. Lagerbarkeit sowie ästhetischen Zwecken. Es ist jedoch auch möglich, solche Komponenten zu verwenden, die eine (konservierende, pflegende) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die folgenden Beispiele dienen dazu, die Erfindung zu beschreiben, selbstverständlich ohne daß beabsichtigt ist, die Erfindung auf diese Beispiele zu beschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

### Beispiel 1:

| | |
|---|---|
| 15 % | Glutaraldehyd |
| 20 % | Dihydroxydioxahexan |
| 5 % | Pyrrolidoncarbonsäure |
| 4 % | Isotridecanolethoxylat |
| 4 % | Fettalkoholpolyglykolether |
| 1 % | Korrosionsschutz |
| 1 % | pH-Regulator |
| Rest | Wasser |

Es wird ein pH-Wert von 4 eingestellt.

### Beispiel 2:

| | |
|---|---|
| 13 % | Didecyldimethylammoniumchlorid |
| 9 % | Dodecylpropylendiamin |
| 1 % | Pyrrolidoncarbonsäure |
| 3 % | Isotridecanolethoxylat |
| 4 % | Fettalkoholpolyglykolether |
| 2 % | Korrosionsschutz |
| 5 % | Glykol |
| 2 % | pH-Regulator |
| 2 % | Komplexbildner |
| Rest | Wasser |

Es wird ein pH-Wert von 9 eingestellt.

### Beispiel 3:

| | |
|---|---|
| 15 % | Glutaraldehyd |
| 1 % | Didecyldimethylammoniumchlorid |
| 1 % | Benzalkoniumchlorid |
| 1 % | Pyrrolidoncarbonsäure |
| 4 % | Isotridecanolethoxylat |
| 4 % | Fettalkoholpolyglykolether |
| 2 % | Korrosionsschutz |
| 6 % | Alkohole |
| 1 % | pH-Regulator |
| Rest | Wasser |

Es wird ein pH-Wert von 4 eingestellt.

### Beispiel 4:

| | |
|---|---|
| 15 % | Glutaraldehyd |
| 1 % | Didecyldimethylammoniumchlorid |
| 1 % | Benzalkoniumchlorid |
| 1 % | Pyrrolidoncarbonsäure |
| 4 % | Isotridecanolethoxylat |
| 2 % | Cocobetainamido Amphopropionat |
| 4 % | Fettalkoholpolyglykolether |
| 2 % | Korrosionsschutz |
| 6 % | Alkohole |
| 1 % | pH-Regulator |
| Rest | Wasser |

Es wird ein pH-Wert von 4 eingestellt.

### Beispiel 5a:

| | |
|---|---|
| 1 % | Pyrrolidoncarbonsäure |
| 6 % | Isotridecanolethoxylat |
| 2 % | Cocobetainamido Amphopropionat |
| 6 % | Fettalkoholpolyglykolether |
| 1 % | Korrosionsschutz |
| 10 % | Glycol |
| Rest | Wasser |

Es wird ein pH-Wert von 4 eingestellt.

### Beispiel 5b:

| | |
|---|---|
| 15 % | Glutaraldehyd |
| 20 % | Dihydroxydioxahexan |
| 4 % | Isotridecanolethoxylat |
| 4 % | Fettalkoholpolyglykolether |
| 1 % | Korrosionsschutz |
| 1 % | pH-Regulator |
| Rest | Wasser |

Mit einem flüssigen Reinigungsmittel gemäß Beispielrezeptur 5a wurde ein Anwendungstest durchgeführt. Dazu wurden 129 Duoendoskope mit einem Reinigungsmittel des Standes der Technik (enzymatischer Reiniger) und 97 Duoendoskope mit dem erfindungsgemäßen Reinigungsmittel jeweils gereinigt und anschließend mit der desinfizierenden Zubereitung gemäß Beispielrezeptur 5b desinfiziert; danach wurde der mikrobiologische Status der Geräte überprüft. Es ergaben sich die folgenden Ergebnisse:

| | eR¹ | Beispiel 3² |
|---|---|---|
| geprüfte Duoendoskope | 129 | 97 |
| mikrobiologisch einwandfrei | 94 | 91 |
| mikrobiologisch nicht einwandfrei | 35 | 6 |

| | | |
|---|---|---|
| ¹ enzymatischer Reiniger | | |
| ² Reinigungsmittel gemäß Beispiel 5 | | |

## Patentansprüche

1. Reinigendes und desinfizierendes System für medizinische Instrumente, **dadurch gekennzeichnet, daß** es
■ mindestens ein nichtionisches Tensid und
■ Pyrrolidoncarbonsäure
■ sowie mindestens ein desinfizierendes Agens
umfaßt.

2. Reinigendes und desinfizierendes System nach Anspruch 1, **dadurch gekennzeichnet, daß** das oder die nichtionischen Tenside gewählt werden aus der Gruppe der Alkylethoxylate, deren Alkylgruppe eine gesättigte oder ungesättigte, gerad- oder verzweigtkettige Alkylgruppe mit 10 bis 18, vorzugsweise 12 bis 14 Kohlenstoffatomen ist.

3. Reinigendes und desinfizierendes System nach einem der Ansprüche 1 oder 2 **dadurch gekennzeichnet, daß** es als nichtionisches Tensid isotridecanolethoxylat und/oder Fettalkoholpolyglykolether enthält.

4. Reinigendes und desinfizierendes System nach einem der Ansprüche 1 bis 3, **dadurch gekennzeichnet, daß** das oder die desinfizierenden Agentien gewählt werden aus der Gruppe der Aldehyde (z.B. Formaldehyd, Glyoxal, Glutaraldehyd), der Phenolderivate (z. B. 2-Biphenylol und p-Chlor-m-kresol (4-Chlor-3-methylphenol), der Alkohole, der quaternären Ammonium-Verbindungen (z. B. Benzalkoniumchlorid, Cetrimoniumbromid, Cetylpyridiniumchlorid (Hexadecylpyridiniumchlorid).

5. Reinigendes und desinfizierendes System nach einem der Ansprüche 1 bis 4, **dadurch gekennzeichnet, daß** es als weitere(n) Bestandteil(e) mindestens ein Amphotensid enthält.

6. Reinigendes und desinfizierendes System nach Anspruch 5, **dadurch gekennzeichnet, daß** das oder die Amphotenside gewählt werden aus der Gruppe der Amphotenside, deren Alkylgruppe eine gesättigte oder ungesättigte, gerad- oder verzweigtkettige Alkylgruppe mit 10 bis 18, vorzugsweise 12 bis 14 Kohlenstoffatomen ist.

7. Reinigendes und desinfizierendes System nach einem der Ansprüche 5 oder 6, **dadurch gekennzeichnet, daß** es als Amphotensid Cocobetainamido Amphopropionat enthält.

8. Reinigendes und desinfizierendes System nach einem der Ansprüche 5 bis 7, **dadurch gekennzeichnet, daß** die Menge an Amphotensid (eine oder mehrere Verbindungen) aus dem Bereich von 1,0 bis 10,0 Gew.-%, vorzugsweise von 2,0 bis 5,0 Gew.-%, jeweils bezogen auf das Gesamtgewicht der Zubereitungen, gewählt wird.

9. Reinigendes und desinfizierendes System nach einem der Ansprüche 1 bis 8, **dadurch gekennzeichnet, daß** es ein oder mehrere nichtionische Tenside in einer Menge von 1,0 bis 20,0 Gew.-%, vorzugsweise von 5,0 bis 15,0 Gew.-%, jeweils bezogen auf das Gesamtgewicht der Zubereitungen, enthält.

10. Reinigendes und desinfizierendes System nach einem der Ansprüche 1 bis 9, **dadurch gekennzeichnet, daß** es die Pyrrolidoncarbonsäure in einer Menge von 0,1 bis 10,0 Gew.-%, vorzugsweise von 0,5 bis 2,0 Gew.-%, jeweils bezogen auf das Gesamtgewicht der Zubereitungen, enthält.

11. Reinigendes und desinfizierendes System nach einem der Ansprüche 1 bis 10, **dadurch gekennzeichnet, daß** es einen pH-Wert zwischen 4 und 10 hat.

12. Reinigendes und desinfizierendes System nach einem der Ansprüche 1 bis 11, **dadurch gekennzeichnet, daß** es in Form eines Konzentrats vorliegt, welches vor der Verwendung mit Wasser zu einer Gebrauchslösung verdünnt wird.

13. Reinigendes und desinfizierendes System, welches so ausgestaltet ist, daß es
A) eine erste Zubereitung in Form eines flüssigen Reinigungsmittels für medizinische Instrumente, welches
• mindestens ein nichtionisches Tensid und
• Pyrrolidoncarbonsäure
enthält,
und
B) eine zweite Zubereitung, welche in einem geeigneten Träger
• mindestens ein desinfizierendes Agens
enthält,
umfaßt.

14. Verwendung von reinigenden und desinfizierenden Systemen, welche
■ mindestens ein nichtionisches Tensid und
■ Pyrrolidoncarbonsäure,
■ sowie mindestens ein desinfizierendes Agens
enthalten, zur Reinigung und Desinfektion medizinischer Instrumente, flexibler Endoskope und medizinischer Oberflächen.

## Claims

1. Cleaning and disinfecting system for medical instruments, **characterized in that** it comprises
■ at least one non-ionic surfactant and
■ pyrrolidonecarboxylic acid
■ and at least one disinfecting agent.

2. Cleaning and disinfecting system according to Claim 1, **characterized in that** the non-ionic surfactant(s) is/are chosen from the group consisting of the alkyl ethoxylates whose alkyl group is a saturated or unsaturated, straight- or branched-chain alkyl group having 10 to 18, preferably 12 to 14, carbon atoms.

3. Cleaning and disinfecting system according to one of Claims 1 or 2, **characterized in that** as the non-ionic surfactant it contains isotridecanol ethoxylate and/or fatty alcohol polyglycol ether.

4. Cleaning and disinfecting system according to one of Claims 1 to 3, **characterized in that** the disinfecting agent(s) is/are chosen from the group consisting of the aldehydes (e.g. formaldehyde, glyoxal, glutaraldehyde), the phenol derivatives (e.g. 2-biphenylol and p-chloro-m-cresol (4-chloro-3-methylphenol), the alcohols, the quaternary ammonium compounds (e.g. benzalkonium chloride, cetrimonium bromide, cetylpyridinium chloride (hexadecylpyridinium chloride).

5. Cleaning and disinfecting system according to one of Claims 1 to 4, **characterized in that** as the further constituent(s) it contains at least one amphosurfactant.

6. Cleaning and disinfecting system according to Claim 5, **characterized in that** the ampho-surfactant(s) is/are chosen from the group consisting of the amphosurfactants whose alkyl group is a saturated or unsaturated, straight- or branched-chain alkyl group having 10 to 18, preferably 12 to 14, carbon atoms.

7. Cleaning and disinfecting system according to one of Claims 5 or 6, **characterized in that** as the amphosurfactant it contains cocobetainamido amphopropionate.

8. Cleaning and disinfecting system according to one of Claims 5 to 7, **characterized in that** the amount of amphosurfactant (one or more compounds) is chosen from the range from 1.0 to 10.0% by weight, preferably from 2.0 to 5.0% by weight, in each case based on the total weight of the preparations.

9. Cleaning and disinfecting system according to one of Claims 1 to 8, **characterized in that** it contains one or more non-ionic surfactants in an amount from 1.0 to 20.0% by weight, preferably from 5.0 to 15.0% by weight, in each case based on the total weight of the preparations.

10. Cleaning and disinfecting system according to one of Claims 1 to 9, **characterized in that** it contains pyrrolidonecarboxylic acid in an amount from 0.1 to 10.0% by weight, preferably from 0.5 to 2.0% by weight, in each case based on the total weight of the preparations.

11. Cleaning and disinfecting system according to one of Claims 1 to 10, **characterized in that** it has a pH of between 4 and 10.

12. Cleaning and disinfecting system according to one of Claims 1 to 11, **characterized in that** it is present in the form of a concentrate which before use is diluted with water to give a use solution.

13. Cleaning and disinfecting system which is equipped such that it comprises
A) a first preparation in the form of a liquid cleaning agent for medical instruments, which contains
■ at least one non-ionic surfactant and
■ pyrrolidonecarboxylic acid,
and
B) a second preparation which, in a suitable carrier,
■ contains at least one disinfecting agent.

14. Use of cleaning and disinfecting systems which contain
■ at least one non-ionic surfactant and
■ pyrrolidonecarboxylic acid,
■ and at least one disinfecting agent
for the cleaning and disinfection of medical instruments, flexible endoscopes and medical surfaces.

## Revendications

1. Système de nettoyage et de désinfection d'instruments médicaux, **caractérisé en ce qu'**il contient:
- au moins un agent tensioactif non ionique,
- de l'acide pyrrolidone carboxylique
- ainsi qu'au moins un agent désinfectant.

2. Système de nettoyage et de désinfection selon la revendication 1, **caractérisé en ce que** le ou les agents tensioactifs non ioniques sont sélectionnés dans le groupe des éthoxylates d'alkyle dont le groupe alkyle est un groupe alkyle saturé ou insaturé à chaîne linéaire ou ramifiée qui compte de 10 à 18 et de préférence de 12 à 14 atomes de carbone.

3. Système de nettoyage et de désinfection selon l'une des revendications 1 ou 2, **caractérisé en ce que** comme agents tensioactifs non ioniques, il contient de l'éthoxylate d'isotridécanol et/ou un polyglycoléther d'alcool gras.

4. Système de nettoyage et de désinfection selon l'une des revendications 1 à 3, **caractérisé en ce que** le ou les agents désinfectants sont sélectionnés dans le groupe des aldéhydes (par exemple le formaldéhyde, le glyoxal, le glutaraldéhyde), des dérivés du phénol (par exemple le 2-biphénylol et le p-chloro-m-crésol(4-chloro-3-méthylphénol), des alcools, des composés d'ammonium quaternaire (par exemple le chlorure de benzalkonium, le bromure de cétrimonium, le chlorure de cétylpyridinium (chlorure d'hexadécylpyridinium).

5. Système de nettoyage et de désinfection selon l'une des revendications 1 à 4, **caractérisé en ce qu'**il contient comme autre(s) composant(s) au moins un agent tensioactif amphotère.

6. Système de nettoyage et de désinfection selon la revendication 5, **caractérisé en ce que** le ou les agents tensioactifs amphotères sont sélectionnés dans le groupe des agents tensioactifs amphotères dont le groupe alkyle est un groupe alkyle saturé ou insaturé à chaîne linéaire ou ramifiée qui compte de 10 à 18 et de préférence de 12 à 14 atomes de carbone.

7. Système de nettoyage et de désinfection selon l'une des revendications 5 ou 6, **caractérisé en ce que** comme agent tensioactif amphotère, il contient un amphopropionate de cocobétaïnamido.

8. Système de nettoyage et de désinfection selon l'une des revendications 5 à 7, **caractérisé en ce que** la quantité en agents tensioactifs amphotères (un ou plusieurs composés) est sélectionnée dans la plage de 1,0 à 10,0% en poids et de préférence de 2,0 à 5,0% en poids, ces pourcentages étant chaque fois calculés par rapport au poids total des préparations.

9. Système de nettoyage et de désinfection selon l'une des revendications 1 à 8, **caractérisé en ce qu'**il contient un ou plusieurs agents tensioactifs non ioniques en quantité de 1 à 20% en poids et de préférence de 5,0 à 15,0% en poids, ces pourcentages étant chaque fois calculés par rapport au poids total des préparations.

10. Système de nettoyage et de désinfection selon l'une des revendications 1 à 9, **caractérisé en ce qu'**il contient l'acide pyrrolidone carboxylique en quantité de 0,1 à 10,% en poids et de préférence de 0,5 à 2,0% en poids, ces pourcentages étant chaque fois calculés par rapport au poids total des préparations.

11. Système de nettoyage et de désinfection selon l'une des revendications 1 à 10, **caractérisé en ce qu'**il a un pH d'une valeur comprise entre 4 et 10.

12. Système de nettoyage et de désinfection selon l'une des revendications 1 à 11, **caractérisé en ce qu'**il se présente sous la forme d'un concentré qui, avant son utilisation, est dilué à l'eau pour obtenir une solution prête à l'emploi.

13. Système de nettoyage et de désinfection configuré de telle sorte qu'il comprend:
A) une première préparation qui présente la forme d'un agent liquide de nettoyage d'instruments médicaux qui contient:
- au moins un agent tensioactif non ionique et
- de l'acide pyrrolidone carboxylique,
et
B) une deuxième préparation qui contient dans un support approprié:
- au moins un agent désinfectant.

14. Utilisation de systèmes de nettoyage et de désinfection qui contiennent
- au moins un agent tensioactif non ionique,
- de l'acide pyrrolidone carboxylique
- ainsi qu'au moins un agent désinfectant
pour le nettoyage et la désinfection d'instruments médicaux, d'endoscopes souples et de surfaces médicales.
